Europäisches Patentamt

European Patent Office

Office européen des brevets

(19)

(11) **EP 0 466 829 B1**

(12) **FASCICULE DE BREVET EUROPEEN**

(45) Date de publication et mention
de la délivrance du brevet:
**24.07.1996 Bulletin 1996/30**

(21) Numéro de dépôt: **90907101.1**

(22) Date de dépôt: **10.04.1990**

(51) Int Cl.$^6$: **C08H 1/06**, A61L 27/00

(86) Numéro de dépôt international:
**PCT/FR90/00253**

(87) Numéro de publication internationale:
**WO 90/12055 (18.10.1990 Gazette 1990/24)**

(54) **PROCEDE DE RETICULATION DU COLLAGENE PAR LE DIPHENYLPHOSPHORYLAZIDE, COLLAGENE RETICULE AINSI OBTENU ET BIOMATERIAUX A BASE DE COLLAGENE AINSI RETICULES**

VERFAHREN ZUR VERNETZUNG VON KOLLAGEN MITTELS DIPHENYLPHOSPHORAZID, DAS SO VERNETZTE KOLLAGEN UND BIOMATERIALIEN AUF DER BASIS VON VERNETZTEM KOLLAGEN

PROCESS FOR CROSS-LINKING OF COLLAGEN BY DIPHENYLPHOSPHORYLAZIDE, THE CROSS-LINKED COLLAGEN OBTAINED THEREBY, AND BIOMATERIALS OF COLLAGEN BASE THUS CROSS-LINKED

(84) Etats contractants désignés:
**AT BE CH DE DK ES FR GB IT LI LU NL SE**

(30) Priorité: **12.04.1989 FR 8904846**

(43) Date de publication de la demande:
**22.01.1992 Bulletin 1992/04**

(73) Titulaire: **COLETICA**
**69007 Lyon (FR)**

(72) Inventeurs:
• **PETITE, Hervé**
**F-69500 Bron (FR)**
• **MENASCHE, Philippe**
**F-75006 Paris (FR)**

• **HUC, Alain**
**F-69110 Ste-Foy-lès-Lyon (FR)**

(74) Mandataire: **Portal, Gérard et al**
**Cabinet Beau de Loménie**
**158, rue de l'Université**
**75340 Paris Cédex 07 (FR)**

(56) Documents cités:
**EP-A- 0 070 021**      **EP-A- 0 301 977**
**FR-A- 2 172 162**      **US-A- 4 755 593**

• **Int. J. Peptide Protein Res., Vol. 22, 1983, J.D. YOUNG et al.: "Synthesis of a Cyclic Fibrin-Like Peptide and its Analysis by Fast Atom Bombardment Mass Spectrometry", pages 374-380**

## Description

La présente invention concerne essentiellement un procédé de réticulation du collagène par le diphénylphosphorylazide, le collagène réticulé ainsi obtenu ainsi que les biomatériaux à base de collagène ainsi réticulés.

On sait que le collagène constitue le tiers des protéines de l'être vivant. Sa faible immunogénicité, son effet sur la croissance cellulaire et ses propriétés mécaniques importantes lui confèrent un intérêt évident en tant que matière première pour les biomatériaux. Cependant, lorsqu'il est implanté, il subit une dégradation plus ou moins rapide selon la forme sous laquelle il se trouve (solutions, éponges, films ou tissus natifs), selon le lieu d'implantation et l'espèce animale. Cette dégradation du collagène est parfois souhaitable (pansements cicatrisants), parfois gênante (bioprothèses valvulaires, vasculaires, etc.).

Par ailleurs, la dégradation du collagène est un processus normal faisant partie de la croissance, du développement et du renouvellement des tissus conjonctifs. Elle fait aussi partie intégrante du processus de cicatrisation. Cette dégradation du collagène fait intervenir un certain nombre d'enzymes, en particulier les collagénases qui sont responsables de l'attaque initiale du collagène natif à pH neutre. Elles cliveraient les trois chaînes peptidiques simultanément. Le clivage a lieu entre les résidus GLY-LEU ou GLY-ILE. Cependant, il est actuellement admis que cette dégradation nécessite les effets coopératifs d'un certain nombre d'enzymes parmi lesquelles la stromélysine, les gélatinases.

Lors de l'utilisation du collagène en tant que biomatériau, il est par conséquent très intéressant de pouvoir moduler la biodégradabilité du collagène en fonction de l'utilisation envisagée. Cette biodégradabilité peut être modulée par au moins deux manières qui consistent soit en l'addition d'inhibiteurs enzymatiques (tels que l'$\alpha$-2-macroglobuline ou la $\beta$-1-anticollagénase), soit encore par l'introduction de liaisons chimiques de réticulation entre les molécules de collagène. Cette seconde méthode est la plus largement utilisée, car elle est plus efficace sur le plan de la résistance à la dégradation enzymatique. Les liaisons de réticulation peuvent être obtenues soit par des méthodes physiques qui ont l'avantage de ne pas introduire d'agent chimique dans le tissu, mais qui s'avèrent peu efficaces, soit encore par des méthodes chimiques qui sont efficaces mais laissent dans le tissu des traces d'agent réticulant. Ainsi, le glutaraldéhyde (en abrégé GTA) est l'agent réticulant le plus largement employé, mais il a malheureusement la propriété de se polymériser en solution. C'est ainsi que, lors de la réticulation du collagène, il y a formation de polymères de GTA qui relargueront au cours du temps des monomères de GTA (qui sont cytotoxiques à des concentrations supérieures à 10-25 ppm) dans les tissus environnants en faisant perdre au collagène une partie de ses propriétés biologiques pour lesquelles il avait été choisi.

Pour éviter l'utilisation de glutaraldéhyde, Les présents inventeurs avaient déjà proposé, dans le document FR-A-8710317 = EP-A-0301977, un procédé de réticulation du collagène par introduction de groupements azides sur les groupements carboxyliques des chaînes latérales du collagène. Dans ce document, la réticulation était effectuée par une estérification des groupements carboxyliques du collagène, puis les esters étaient successivement transformés en hydrazides, puis en acylazides. Les acylazides réagissaient finalement en milieu basique avec les fonctions aminées des chaînes latérales du collagène pour donner des liaisons de type peptidique. Ce procédé, bien que très innovant, présente l'inconvénient d'être long puisque la réticulation du collagène nécessite 8 jours et est peu commode à utiliser à l'échelle industrielle, ce délai étant difficilement compatible avec une exploitation industrielle.

Les présents inventeurs ont poursuivi leurs recherches afin de simplifier la méthode de réticulation tout en n'introduisant pas d'agent réticulant dans le matériau fini et en obtenant un degré de réticulation du collagène équivalent à celui obtenu par le procédé décrit dans le document FR-A-8710317 = EP-A-0301977.

Le diphénylphosphorylazide a été décrit comme agent de couplage pour réaliser la synthèse de peptides dans le document FR-2172162. Il est également décrit comme agent pour réaliser la préparation de peptides cycliques à partir de peptides linéaires dans les documents EP-A-0070021 et Int.J. Peptide Protein Research. 22,983,374-380.

Ainsi, la présente invention a pour but de résoudre le nouveau problème technique consistant en la fourniture d'un procédé de réticulation du collagène simplifié tout en n'introduisant pas d'agent réticulant dans le matériau fini et en obtenant un degré de réticulation du collagène équivalent à celui obtenu par les procédés antérieurement décrits, et notamment celui décrit dans le document FR-A-8710317 = EP-A-0301977.

La présente invention a encore pour but de résoudre le nouveau problème technique énoncé ci-dessus avec une durée de réticulation radicalement réduite.

La présente invention résout pour la première fois les problèmes techniques énoncés ci-dessus d'une manière extrêmement simple, reproductible, dont les paramètres du procédé peuvent être variés à volonté, de manière à régler à volonté le degré de réticulation du collagène en fonction des utilisations souhaitées, et à un faible coût.

Ainsi, selon un premier aspect, la présente invention fournit un procédé de réticulation du collagène du type comprenant la formation de liaisons amides par l'intermédiaire de groupements acylazides, caractérisé en ce qu'on fait réagir le collagène avec le diphènylphosphorylazide (DPPA).

Selon un mode de réalisation avantageux du procédé selon l'invention, la réaction avec le DPPA a lieu dans un milieu solvant non aqueux. De préférence, ce solvant non aqueux est constitué par du diméthylformamide (DMF).

Selon une variante de réalisation avantageuse, la concentration de DPPA est comprise entre 0,0125% et 1,50% en volume/volume et encore de préférence entre 0,25 et 0,7%.

Selon une variante de réalisation avantageuse du procédé selon l'invention, la réaction avec le DPPA est réalisée par incubation à une température d'incubation comprise entre environ 0 et 10°C, de préférence environ 4°C, pendant une durée d'incubation de quelques heures à environ 1 jour, de préférence environ 1 jour.

Selon une caractéristique particulièrement avantageuse du procédé selon l'invention, après réaction du collagène avec le DPPA, on réalise au moins un rinçage pour éliminer le DPPA, puis on introduit le collagène comportant des groupements acylazides dans une solution de tampon borate.

Selon un mode de réalisation avantageux du procédé selon l'invention, on réalise une incubation du collagène comportant des groupements acylazides dans le tampon borate, avantageusement à une température comprise entre 0 et 10°C, encore de préférence d'environ 40°C, pendant une durée d'incubation de quelques heures à 1 jour, de préférence environ 1 jour.

Selon un mode de réalisation avantageux, le tampon borate est à un pH environ égal à 8,9.

Selon encore un autre mode de réalisation avantageux du procédé selon l'invention, le collagène utilisé comme matériau de départ et mis à réagir avec le DPPA est sous forme de gel, de film ou de tissu naturel par exemple péricarde ou greffon vasculaire, d'une éponge, d'un tube, ou d'un fil de collagène.

Selon un autre mode de réalisation avantageux, le collagène utilisé comme matériau de départ est sous forme d'un tissu naturel qui, après réticulation au DPPA, est conservé dans une solution d'éthanol à 70°.

Selon un autre mode de réalisation avantageux, après réticulation du collagène avec le DPPA, on réalise une stérilisation, par exemple stérilisation d'un film de collagène aux rayons gamma.

Sans qu'on puisse connaître avec certitude le mécanisme réactionnel mis en jeu lors de la réticulation du collagène par le DPPA, on peut, en raisonnant par analogie avec le mécanisme réactionnel proposé pour la formation de liaison amide par le DPPA - sur les acides carboxyliques, proposer le mécanisme réactionnel représente à la figure annexée. On observe que l'anion carboxylique (a), qui est fourni par les groupements carboxyliques latéraux de de l'acide aspartique ou glutamique des chaines peptidiques du collagène, peut attaquer l'atome de phosphore du DPPA (b) pour donner essentiellement un composé phosphoré pentacovalent (c). Ensuite, il se produit une migration du groupement azide de l'atome de phosphore vers l'atome de carbone carboxylique par un réarrangement de type substitution nucléophile interne.

Finalement, l'acylazide formé (d) va réagir avec les groupements aminés latéraux de la lysine et de l'hydroxylysine des chaînes peptidiques du collagène pour donner une liaison de type amide.

On constate ainsi que l'invention permet de simplifier d'une manière totalement inattendue le procédé de réticulation du collagène, ce procédé étant extrêmement simple, peu coûteux, et ne nécessitant qu'une durée relativement faible de réticulation, ce qui représente un avantage technique remarquable, déterminant pour un homme de l'art par rapport aux techniques antérieures.

D'autres buts, caractéristiques et avantages de l'invention apparaîtront clairement à la lumière de la description explicative qui va suivre faite en référence à plusieurs exemples de réalisation donnés simplement à titre d'illustration et qui ne sauraient donc en aucune façon limiter la portée de l'invention.

Dans les dessins,

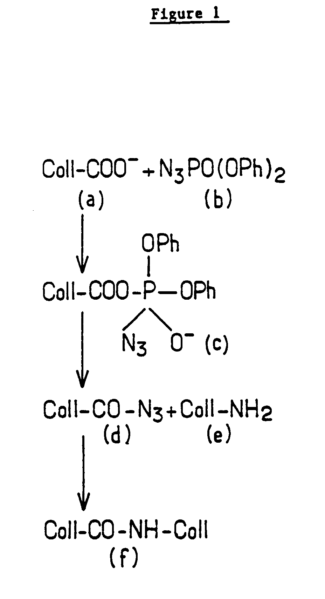

- la figure 1 représente une hypothèse de mécanisme réactionnel de la réaction de réticulation du collagène par réaction du diphénylphosphorylazide sur les groupements carboxyliques du collagène;
- la figure 2 représente l'évolution du pourcentage de réticulation du collagène, appelé RTP, mesuré à partir de la température du pic de dénaturation (TP) du collagène, sur du péricarde de veau en fonction de la concentration en DPPA ; et
- la figure 3 représente l'évolution du pourcentage de réticulation RTP sur des films de collagène en fonction de la concentration en DPPA.

EXEMPLE 1

Réticulation par le DPPA du péricarde de veau.

a) Préparation du matériau de départ.

On prélève aux abattoirs des péricardes de veau moins d'une heure après leur mise à mort. Ces péricardes sont alors dégraissés et lavés dans une solution de NaCl 0,9%. Ils sont ensuite découpés à l'emporte-pièce en pastilles de 1 cm$^2$.

b) Réticulation du péricarde :

Cette réaction peut être résumée comme suit :

Quatre pastilles de péricarde, préalablement rincées dans 10 ml de DMF pur durant 5 min afin de débarrasser le tissu de son eau, sont incubées pour 24 h à 4°C dans 10 ml d'une solution de DMF contenant 0,75% de DPPA (concentration exprimée en volume/volume). Le tissu est ensuite débarrassé du DPPA par rinçage dans 10 ml de solution de DMF. Le DMF est ensuite éliminé par rinçage dans 10 ml d'une solution de tampon borate pH 8,9 (tétraborate de sodium 0,04 M, acide borique 0,04 M). Le tissu est finalement incubé pour une nuit dans un tampon borate pH 8,9. Le tissu est alors par exemple conservé dans une solution d'éthanol à 70°.

EXEMPLE 2

Réticulation par le DPPA d'un film de collagène

a) Préparation du matériau de départ.

Un gel est préparé à partir de peaux de veaux préalablement lavées et épilées par un mélange chaux-sulfure. Elles sont ensuite déchaulées dans un bain contenant du chlorure d'ammonium (2%) et du métabisulfite de sodium. Elles sont ensuite neutralisées, puis les sels sont éliminés par deux lavages à l'eau. Elles sont alors broyées, puis lavées par du tampon phosphate pH 7,8 (dihydrogénophosphate de potassium 0,78 g/l et monohydrogénophosphate disodique 21,7 g/l). Le phosphate est ensuite éliminé par deux lavages successifs à l'eau permutée. Le broyat est alors acidifié par une solution d'acide acétique à 10%, la quantité d'acide acétique étant de 5% par rapport au collagène. Le broyat est alors malaxé afin d'obtenir une pâte homogène. Cette pâte est alors diluée pour obtenir un gel ayant une concentration de 0,7% en collagène. Le gel est alors mis dans de petits moules en Téflon, puis laissé évaporer. Le film ainsi obtenu est ensuite découpé à l'emporte-pièce en pastille de 1 cm$^2$.

b) Réticulation du film :

Cette réaction peut être résumée comme suit :

Quatre pastilles de films de 1 cm$^2$ de surface sont incubées pour 24 h à 4° dans 10 ml d'une solution de DMF contenant 0,25% de DPPA (concentration exprimée en volume/volume). Le film est ensuite débarrassé du DPPA par rinçage dans 10 ml de solution de DMF. Le DMF est ensuite éliminé par rinçage dans 10 ml d'une solution tampon borate pH 8,9 (tétraborate de sodium 0,04 M, acide borique 0,04 M). Les films sont finalement incubés pendant une nuit dans un tampon borate pH 8,9. Ils sont alors essorés sur un papier-filtre, puis séchés à l'air libre. Ils peuvent être ensuite stérilisés par exemple aux rayons gamma.

De la même façon, on peut réticuler des éponges, des tubes, des fils de collagène, etc.

EXEMPLE 3

A. Influence de la concentration en DPPA sur la réticulation du péricarde de veau.

Une première partie du travail a consisté à étudier l'influence de la concentration en DPPA sur le degré de réticulation du péricarde. Pour cela, le péricarde est incubé dans des solutions de DPPA de concentrations variant entre 0,0125 % et 1,5 % (volume/volume).

La mesure du degré de réticulation du collagène est réalisée par analyse calorimétrique à balayage. Cette technique consiste à mesurer, lors d'une montée linéaire de la température, la différence d'énergie qu'il faut fournir à l'échantillon et à une référence pour les maintenir à une température identique. Lorsque le collagène se dénature, un pic d'absorption de chaleur apparaît sur l'enregistreur. On définit ainsi la température de début de dénaturation (TD), la température du pic de dénaturation (TP) et la température de fin de dénaturation (TF).

Afin de calculer un pourcentage de réticulation R du collagène, on effectue le calcul suivant :

$$R = \frac{(TX-TI)}{(TM-TI)} \times 100$$

TM : température de dénaturation maximum qu'il est possible d'obtenir lorsqu'on réticule le collagène par le DPPA; en l'occurrence, pour le cas présent, elle correspond à la température obtenue avec une concentration en DPPA ([DPPA]) de 0,75%.

TI : température de dénaturation obtenue sur le tissu non traité.

TX : température de dénaturation obtenue sur le tissu avec une concentration en DPPA X.

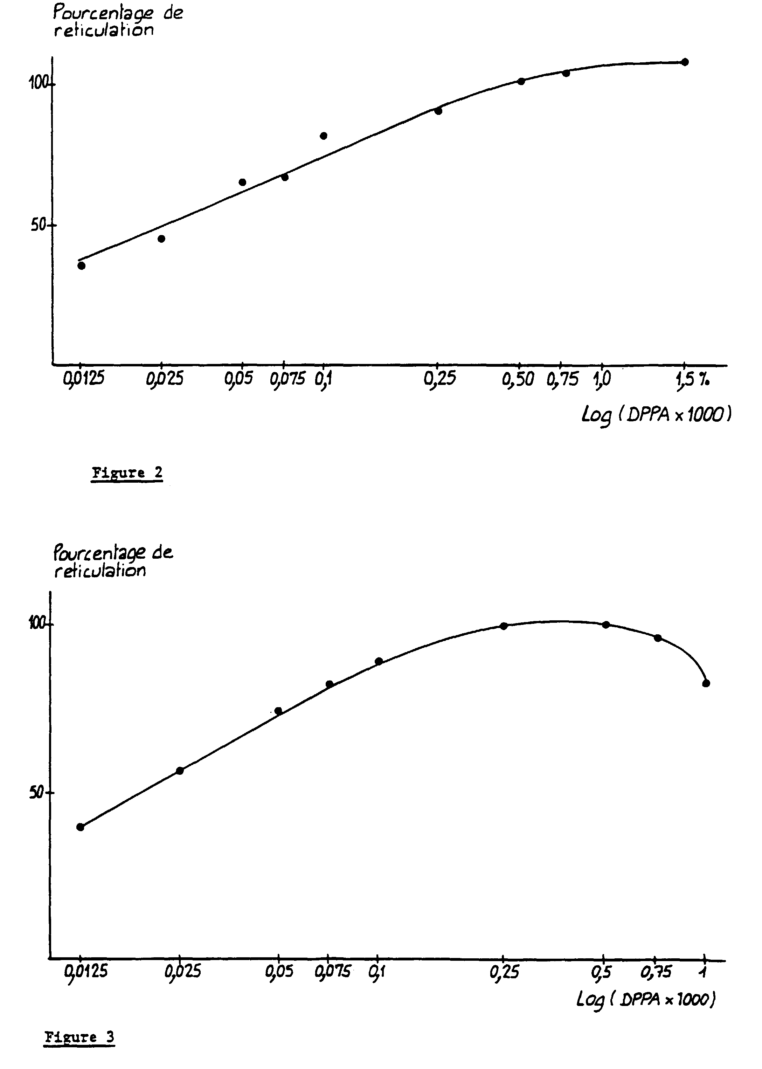

R est le % de réticulation calculé à partir de TP, la température du pic de dénaturation, et est appelé RTP. L'évolution de RTP en fonction du Log([DPPA]x1000) est représentée sur la figure 2. Les inventeurs déterminent ainsi qu'entre 0,0125% et 0,50% l'évolution de RTP en fonction du Log(IDPPA x1000) est linéaire et qu'à partir de 0,5-0,75% on obtient une réticulation maximum et constante quelle que soit la concentration en DPPA.

Si l'on compare la température de dénaturation obtenue avec le DPPA 0,75% ou 0,5% à celle obtenue avec le procédé proposé dans le brevet n° 8710317 et à celle du GTA 0,6% (traitement classique des biprothèses valvulaires), on constate que ces valeurs ne sont pas significativement différentes (tableau I).

B. Influence de la concentration en DPPA sur la réticulation de film de collagène.

Nous avons étudié l'influence de la concentration en DPPA sur le degré de réticulation du film de collagène. Pour cela, le film est incubé dans des solutions de DPPA de concentrations variant entre 0,0125% et 1,0% (volume/volume).
Tout comme avec le péricarde de veau, on calcule l'évolution de RTP en fonction du Log([DPPA]x1000) (figure 3). Les inventeurs déterminent ainsi qu'entre 0,0125% et 0,10% l'évolution de RTP en fonction du Log([DPPA]x1000) est linéaire. Un maximum de réticulation étant obtenu avec des concentrations en DPPA de 0,25%-0,5%.
Si l'on compare la température de dénaturation obtenue avec le DPPA 0,15% ou 0,5% à celle obtenue avec le procédé proposé dans le brevet n° 8710317 et à celle du GTA 0,6% (traitement classique des bioprothèses valvulaires), on constate que ces valeurs ne sons pas significativement différentes (tableau I).

C. Dosage du DPPA après réticulation du péricarde.

Afin de déterminer le devenir du DPPA après réticulation, un dosage du DPPA par l'intermédiaire de son groupement phosphore est réalisé.
Dans un premier temps, on dose le phosphore sur le matériau traité par le DPPA à différentes concentrations sans effectuer de rinçage. Le matériau traité est simplement séché au four à 110°C. Le dosage du phosphore est réalisé par spectrométrie d'émission plasma après minéralisation par une solution d'acides perchlorique et nitrique (2/3 / 1/3 en volume/volume). Les résultats sont exprimés en pourcentage de phosphore par rapport au poids de tissu sec. Les résultats sont consignés dans le tableau II.
Dans un second temps, on dose le phosphore sur le matériau traité par le DPPA à des concentrations de 0,5% et 1,0% après avoir rincé le tissu soit dans du tampon borate seul, soit dans du DMF, puis du tampon borate. Les résultats sont consignés dans le tableau III.

TABLEAU I

| Comparaison des températures de dénaturation de péricarde de veau ou de film de collagène traités par le GTA, les acylazides selon le procédé du brevet n° 8710317, le DPPA ou non traité. | | | |
|---|---|---|---|
| | Température de dénaturation, °C | | |
| | TD | TP | TF |
| Péricarde frais | 64,30 | 67,30 | 81,30 |
| Péricarde GTA 0,6% | 82 | 85,30 | 93,80 |
| Péricarde azide | 78 | 81,40 | 89,10 |
| Péricarde DPPA 0,5% | 78,50 | 81,40 | 91,70 |
| Péricarde DPPA 0,75% | 79,20 | 82 | 89,90 |
| Film non traité | 39,15 | 49,12 | 66,10 |
| Film GTA 0,6% | 71,10 | 79,10 | 86,40 |
| Film azide | 65,50 | 74,40 | 83 |
| Film DPPA 0,25% | 69,90 | 72,70 | 80,50 |
| Film DPPA 0,50% | 70,30 | 72,60 | 79,20 |

TABLEAU II

| | | | | | | |
|---|---|---|---|---|---|---|
| Pourcentage de phosphore résiduel dosé directement après traitement du péricarde par le DPPA à des concentrations allant de 0% à 1,5%. Aucun rinçage n'étant effectué après traitement par le DPPA. | | | | | | |
| [DPPA] % | 0,00 | 0,25 | 0,50 | 0,75 | 1,00 | 1,50 |
| Phosphore % | 0,1±0,0 | 1,0±0,1 | 1,4±0,2 | 1,7±0,2 | 1,7±0,2 | 2,2±0,1 |

TABLEAU III

| | | | | |
|---|---|---|---|---|
| Dosage du phosphore résiduel après traitement du péricarde par le DPPA à des concentrations de 1,0% et 0,5%. La quantité de phosphore déterminée après 3 rinçages du tissu dans le borate pour une concentrations de 0,5% n'est pas différente de celle déterminée pour un tissu incubé dans le DMF sans agent réticulant (cf. tableau II). | | | | |
| Concentration en DPPA | 0 rinçage DMF 3 rinçages borate | 3 rinçages DMF 3 rinçages borate | 5 rinçages DMF 3 rinçages borate | 8 rinçages DMF 3 rinçages borate |
| 1,0% | 0,22 ± 0,02 | 0,23 ± 0,06 | 0,17 ± 0,03 | 0,18 ± 0,01 |
| 0,5% | 0,09 ±0,06 | 0,04 ± 0,05 | 0,09 ± 0,03 | 0,1 ± 0,06 |

Il apparaît qu'avec une concentration en DPPA de 0,5% le phosphore résiduel, meme après simplement trois rinçages avec du tampon borate, est équivalent à celui trouvé pour un péricarde incubé dans le DMF sans agent réticulant (respectivement 0,09% et 0,1%).

Ainsi, il apparaît que l'agent réticulant ne subsiste pas dans le tissu après réticulation. Ainsi, ce nouveau procédé apparaît tout à fait innovant. Il permet, tout comme le procédé proposé dans le document FR-A-8710317, une réticulation du collagène sans introduire d'agent réticulant de façon définitive et, de plus, il est d'une utilisation nettement facilitée sur le plan industriel.

Naturellement, l'invention comprend tous les moyens constituant des équivalents techniques des moyens décrits ainsi que leurs diverses combinaisons.

**Revendications**

1. Procédé de réticulation du collagène du type comprenant la formation de liaisons amides par l'intermédiaire de groupements acylazides, caractérisé en ce qu'on fait réagir le collagène avec le diphénylphosphorylazide (DPPA).

2. Procédé selon la revendication 1, caractérisé en ce que la réaction avec le DPPA a lieu dans un milieu solvant non aqueux.

3. Procédé selon la revendication 2, caractérisé en ce que le solvant non aqueux est le diméthylformamide.

4. Procédé selon l'une des revendications 1 à 3, caractérisé en ce que la concentration en DPPA est comprise entre 0.0125% et 1.5% en volume/volume, de préférence entre 0.25 et 0.70%.

5. Procédé selon l'une des revendications 1 à 4, caractérisé en ce que la réaction est réalisée par incubation à une température comprise entre - 0 et 10°C, de préférence à environ 4°C, la durée d'incubation étant de préférence comprise entre quelques heures et 1 jour, de préférence d'environ 1 jour.

6. Procédé selon l'une des revendication 1 à 5, caractérisé en ce qu'après réaction avec le DPPA on réalise au moins un rinçage pour éliminer le DPPA, puis on introduit le collagène comportant des groupements acylazides dans une solution de tampon borate.

7. Procédé selon la revendication 6, caractérisé en ce q'uon réalise une incubation à une température comprise entre 0 et 10°C, de préférence environ 4°C, dans le tampon borate.

8. Procédé selon la revendication 7, caractérisé en ce que le tampon borate est à un pH égal à environ 8,9.

**9.** Procédé selon l'une des revendications 1 à 8, caractérisé que le collagène utilisé comme matériau de départ est sous forme de gel, de film ou de tissu naturel, par exemple péricarde ou greffon vasculaire, d'une éponge, d'un tube ou d'un fil de collagène.

**10.** Procédé selon l'une des revendications 1 à 9, caractérisé en ce que le collagène de départ est un tissu naturel qui, après réticulation au DPPA, est conservé dans une solution d'éthanol à 70°.

**11.** Procédé selon l'une des revendications 1 à 9, caractérisé en ce que après réalisation du collagène au DPPA, on réalise une stérilisation, par exemple stérilisation d'un film de collagène aux rayons gamma.

**Patentansprüche**

**1.** Verfahren zur Vernetzung von Kollagen, welches die Bildung von Amid-Bindungen mit Hilfe von Acylazid-Gruppen umfaßt, dadurch gekennzeichnet, daß Kollagen mit Diphenylphosphorylazid (DPPA) umgesetzt wird.

**2.** Verfahren nach Anspruch 1, dadurch gekennzeichnet, daß das Umsetzen mit DPPA in einem nicht-wässerigen Lösungsmittelmedium stattfindet.

**3.** Verfahren nach Anspruch 2, dadurch gekennzeichnet, daß das nicht-wässerige Lösungsmittel Dimethylformamid ist.

**4.** Verfahren nach einem der Ansprüche 1 bis 3, dadurch gekennzeichnet, daß die DPPA-Konzentration zwischen 0,0125 % und 1,5 % V/V, vorzugsweise zwischen 0,25 und 0,70 %, beträgt.

**5.** Verfahren nach einem der Ansprüche 1 bis 4, dadurch gekennzeichnet, daß die Reaktion durch Inkubation bei einer Temperatur zwischen 0 und 10°C, vorzugsweise bei ungefähr 4°C, durchgeführt wird, wobei die Dauer der Inkubation vorzugsweise zwischen einige Stunden bis 1 Tag, vorzugsweise ungefähr 1 Tag, beträgt.

**6.** Verfahren nach einem der Ansprüche 1 bis 5, dadurch gekennzeichnet, daß nach dem Umsetzen mit DPPA zumindest eine Spülung zur Eliminierung von DPPA durchgeführt und dann Acylazid-Gruppen umfassendes Kollagen in eine Borat-Pufferlösung eingebracht wird.

**7.** Verfahren nach Anspruch 6, dadurch gekennzeichnet, daß eine Inkubation bei einer Temperatur zwischen 0 und 10°C, vorzugsweise bei ungefähr 4°C, in Borat-Puffer durchgeführt wird.

**8.** Verfahren nach Anspruch 7, dadurch gekennzeichnet, daß der Borat-Puffer einen pH von ungefähr 8,9 aufweist.

**9.** Verfahren nach einem der Ansprüche 1 bis 8, dadurch gekennzeichnet, daß das als Ausgangsmaterial verwendete Kollagen in Form eines Gels, Films oder natürlichen Gewebes, beispielsweise Perikard oder Gefäßtransplantat, Schwamms, Schlauchs oder einer Kollagenfaser vorliegt.

**10.** Verfahren nach einem der Ansprüche 1 bis 9, dadurch gekennzeichnet, daß das Ausgangs-Kollagen ein natürliches Gewebe ist, das nach Vernetzung mit DPPA in einer Ethanol-Lösung bei 70° konserviert wird.

**11.** Verfahren nach einem der Ansprüche 1 bis 9, dadurch gekennzeichnet, daß nach der Vernetzung von Kollagen mit DPPA eine Sterilisation, beispielsweise eine Sterilisation eines Kollagenfilms mit Gamma-Strahlen, durchgeführt wird.

**Claims**

**1.** Process for cross-linking collagen of the type comprising the formation of amide bonds by using acylazide groups, characterized in that the collagen is reacted with diphenylphosphorylazide (DPPA).

**2.** Process according to claim 1, characterized in that the reaction with DPPA takes place in a non-aqueous solvent medium.

3. Process according to claim 2, characterized in that the non-aqueous solvent is dimethyl formamide.

4. Process according to one of claims 1 to 3, characterized in that the DPPA concentration is comprised between 0.0125% and 1% in volume/volume, preferably between 0.25 and 0.70%.

5. Process according to one of claims 1 to 4, characterized in that the reaction is obtained by incubation at a temperature comprised between 0 and 10°C, preferably at about 4°C, the incubation period being preferably comprised between a few hours and one day, preferably about one day.

6. Process according to one of claims 1 to 5, characterized in that after reaction with DPPA, at least one rinse is carried out to eliminate the DPPA, then the collagen containing acylazide groups is introduced into a solution of borate buffer.

7. Process according to claim 6, characterized in that an incubation is effected at a temperature comprised between 0 and 10°C, preferably about 4°C, in the borate buffer.

8. Process according to claim 7, characterized in that the borate buffer has a pH equal to about 8.9.

9. Process according to one of claims 1 to 8, characterized in that the collagen used as starting material is in the form of gel, of film or of natural tissue, such as pericardium or vascular graft, of a sponge, of a tube or of a collagen yarn.

10. Process according to one of claims 1 to 9, characterized in that the starting collagen is a natural tissue which, after cross-linking with DPPA is preserved in a solution of ethanol at 70°C.

11. Process according to one of claims 1 to 9, characterized in that after cross-linking the collagen with DPPA, a sterilization is carried out for example a sterilization of a collagen film using gamma rays.

Figure 1

$$Coll\text{-}COO^{-} + N_3PO(OPh)_2$$
$$(a) \qquad\qquad (b)$$

$$\downarrow$$

$$\begin{array}{c} OPh \\ | \\ Coll\text{-}COO\text{-}P\text{---}OPh \end{array}$$

$$N_3 \quad O^{-} \ (c)$$

$$\downarrow$$

$$Coll\text{-}CO\text{-}N_3 + Coll\text{-}NH_2$$
$$(d) \qquad\qquad (e)$$

$$\downarrow$$

$$Coll\text{-}CO\text{-}NH\text{-}Coll$$
$$(f)$$

Figure 2

Figure 3